# FASCICULE DE BREVET EUROPEEN

(11) **EP 3 576 666 B1**
(45) Date de publication et mention de la délivrance du brevet: **20.11.2024**
(21) Numéro de dépôt: 18706411.8
(22) Date de dépôt: 31.01.2018
(51) Int. Cl.: A61B 90/00, A61B 34/20, A61F 2/46

(54) **DISPOSITIF D'ASSISTANCE A LA POSE D'UNE PROTHESE DE HANCHE DE GENOUX OU D'ÉPAULE**
VORRICHTUNG ZUR UNTERSTÜTZUNG EINES CHIRURGEN BEI DER EINPASSUNG EINER PROTHESE FÜR EINE HÜFTE, EINE SCHULTER, ODER EIN KNIE
DEVICE FOR HELPING A SURGEON FIT A PROSTHESIS, FOR A HIP, SHOULDER OR KNEE

(30) Priorité: 01.02.2017 FR 1770103
(43) Date de publication de la demande: 11.12.2019
(73) Titulaire: Cazal, Laurent, 87000 Limoges (FR)
(72) Inventeur: Cazal, Laurent, 87000 Limoges (FR)
(74) Mandataire: Aquinov
(86) Numéro de dépôt international: PCT/EP2018/052388
(87) Numéro de publication internationale: WO 2018/141787

(56) Documents cités:
- WO-A1-2012/084739
- WO-A1-2015/164402
- WO-A1-2015/172021
- DE-A1- 102015 212 352
- US-A1- 2012 157 887
- US-A1- 2016 175 064

## Description

La présente invention porte sur un dispositif tel que défini dans la revendication 1. Toutes les méthodes divulguées dans cette description ne font pas partie de l'invention.

Les interventions sur des articulations notamment sont nombreuses et courantes. Il existe différents protocoles et différentes voies d'abord.

C'est ainsi qu'il existe une voie d'abord avec luxation avant résection de la tête, celle qui est choisie pour la présente description.

Il existe d'autres voies d'abord notamment celle dite antérieure ou la résection s'effectue in situ, l'articulation non luxée. Les étapes chirurgicales sont différentes mais les procédé et dispositif sont parfaitement applicables.

C'est ainsi que si l'on se focalise sur l'arthroplastie de la hanche par exemple, intervention qui consiste à remplacer l'intégralité de l'articulation par une prothèse condylienne côté bassin et une prothèse fémorale côté fémur, on sait que cette opération est courante.

Elle se déroule en deux étapes, préopératoire et chirurgicale avec un suivi durant toute l'intervention grâce au procédé selon la présente invention, suivie d'une troisième étape consistant en une visite de contrôle.

La première étape préopératoire permet de confirmer la nécessité de l'intervention, de définir une gamme de prothèses susceptibles d'être posées, la décision finale étant prise au cours de l'intervention et de confirmer a priori les zones impactées.

L'arthrose invalidante est une indication commune pour l'arthroplastie.

Les prothèses ont fait des progrès tant pour la partie cotyle que pour la partie prothèse fémorale et le matériel ancillaire a également été grandement amélioré.

Les protocoles chirurgicaux sont également parfaitement définis tout comme les protocoles de rééducation.

Il n'en reste pas moins que l'intervention est laissée pour une très grande part à la dextérité et aux compétences du chirurgien ainsi qu'à son expérience.

Or, quel que soit le type d'intervention orthopédique, quel que soit le protocole utilisé, le chirurgien doit réséquer au moins une partie osseuse, placer au moins une prothèse en lieu et place de la partie réséquée et repositionner ladite prothèse de façon fonctionnelle. En lieu et place d'une résection, il est possible que l'intervention consiste en un resurfaçage de la partie concernée. Les besoins de repérage dans l'espace restent identiques et le procédé avec son dispositif permet une application dans les deux cas.

En effet, le chirurgien travaille en 3 dimensions car les positions des différentes parties naturelles ou artificielles sont positionnées dans l'espace.

Dans le cas de l'arthroplastie, qui est le cas pris en exemple, cela devient encore plus complexe car il y a par exemple une résection de la tête de fémur et un resurfaçage du cotyle. Dans tous les cas, le chirurgien a besoin de se repérer dans l'espace pour déterminer les profondeurs et profils à réaliser tout en sachant jusqu'où il doit aller pour que les deux parties prothétiques puissent coopérer entre elles dans les mêmes conditions qu'avant l'intervention.

Malgré tous les protocoles existants, malgré les matériels ancillaires utilisés, le résultat d'une telle intervention peut conduire à des problèmes de longueurs de membre inférieur modifiées, obligeant le patient à compenser par des semelles orthopédiques.

Il peut aussi se produire des décalages d'orientation angulaire en abduction ou en antéversion conduisant à des amplitudes de mouvements plus limitées donc à une qualité de marche dégradée.

Très important également, le respect de l'offset est une condition pour ne pas perturber la biomécanique du membre opéré. Ce respect est d'autant plus important que les muscles et enveloppes tissulaires adjacents sont directement impliqués.

De même, si le chirurgien constate une dégradation supérieure d'une partie osseuse le conduisant à modifier le protocole prévu, ceci après résection d'une partie osseuse par exemple, il devient difficile pour le chirurgien de pouvoir se repérer, tant en déport et longueur qu'en orientation.

Il est nécessaire de respecter la longueur finale du membre inférieur car au-delà de 1 cm de différence entre les deux membres inférieurs, le patient ressent un inconfort certain sans compter les dissymétries des efforts lors de la marche, pouvant provoquer des usures prématurées des prothèses et/ou des compensations générant des troubles morpho squelettiques.

Dans le cadre de ces assistances lors des interventions, on connaît de l'art antérieur la demande de brevet US 2012157887 qui décrit un matériel avec des capteurs implantés pour permettre des mesures chirurgicales par rapport à l'anatomie du patient, au cours de l'intervention.

Ainsi, un capteur maître est implanté sur le squelette du patient muni de moyens optiques, le bassin dans le cas d'une intervention sur la hanche et un support d'essai de gabarit de prothèse est muni également de moyens optiques. L'un est émetteur et l'autre est récepteur pour permettre un alignement et un positionnement adaptés. L'un des capteurs comprend aussi une centrale inertielle pour améliorer le positionnement angulaire.

Une unité avec processeurs et logiciel assure le traitement des informations optiques et inertielles reçues pour afficher une information visuelle sur un support.

Chaque capteur travaille par faisceau optique et possède une source interne pour le fonctionnement, rechargeable, filaire, toutes les solutions étant envisagées.

De tels capteurs sont de grandes dimensions et doivent être réglés pour vérifier les alignements.

L'utilisation du dispositif reste complexe.

Il existe des agencements avec des capteurs passifs, qui renvoient un signal par exemple optique et une caméra déduit la position du capteur concerné.

Ces méthodes ne permettent pas au chirurgien de visualiser virtuellement, en direct et en continu, les mouvements, les positionnements des différents éléments au cours même de l'intervention, en superposition avec les parties concernées du patient.

La demande de brevet US 2012/157887 A1 divulgue un système pour les opérations de remplacement d'une hanche, le système utilisant deux marqueurs, un sur la hanche et un sur le fémur du patient. Les demandes de brevet WO 2012/084739, WO 2015/164402 et WO 2015/172021 divulguent des systèmes chirurgicaux à base de réalité augmentée. Les demandes de brevet DE 10 2015 212352 et US 2016/175064 divulguent des systèmes de navigation chirurgicale basés sur des marqueurs a code QR.

Le dispositif selon la présente invention vise à recourir à la réalité augmentée afin de permettre une superposition directe d'une image virtuelle affichée par un moyen de visualisation tel que des lunettes de réalité augmentée, sur une vue d'une forme réelle en mouvement. En l'occurrence, le but est de positionner la forme réelle avec les prothèses dans la même configuration que la forme virtuelle enregistrée, soit sous forme de repères soit sous forme complète en cas d'images virtuelles complètes, en disposant des informations dimensionnelles en temps réel.

Le dispositif selon la présente invention permet de calculer les différentiels entre réel et virtuel d'au moins une prothèse par rapport à l'os qui la reçoit et de permettre un positionnement relatif de chacune des au moins une prothèse par rapport à un autre os ou à une autre prothèse.

Pour la suite de la description, l'application retenue est celle d'une arthroplastie totale de hanche, avec un protocole avec luxation préalable et résection postérieure, car elle fait intervenir deux prothèses chacune à positionner en dimensions et en orientations par rapport à l'os qui la reçoit, à savoir le bassin d'une part et le fémur d'autre part de façon à permettre un positionnement adapté entre lesdites deux prothèses entre elles.

C'est ainsi que :
- la prothèse condylienne doit respecter des plages angulaires limitées d'abduction et d'antéversion mais aussi une profondeur d'implantation afin que le centre de rotation après intervention reste inchangé ou à proximité immédiate du centre de rotation naturel avant intervention.
- La prothèse fémorale doit respecter une pénétration fémorale adaptée pour, après intervention, permettre une superposition du centre de rotation de la tête fémorale de la prothèse avec le centre de rotation de la prothèse condylienne et donc avec le centre de rotation initial avant intervention,
de façon que la conjonction de ces positionnements conduisent à un respect de la longueur du membre inférieur et à un respect de l'offset.

Les méthodes actuelles sont complexes à mettre en oeuvre et nécessitent des calculs, des mesures et bien que toutes les précautions soient prises, le problème le plus fréquent reste celui du différentiel de longueur du membre inférieur après intervention par rapport au membre inférieur avant intervention.

Les paramètres intervenants dans cette problématique sont nombreux, creusement non adapté pour l'intégration de la prothèse cotyloïdienne, impaction de la prothèse fémorale dans le fémur, orientations des éléments prothétiques et offset du membre opéré. Aussi, toute assistance et toute aide pratique est signe de progrès à condition que cette aide soit apportée sans perturbation du champ de l'intervention, soit naturelle pour le praticien qui n'a pas à effectuer des mouvements différents de ceux qu'il pratique habituellement, le tout sans inconfort et en laissant le praticien totalement libre de ses gestes.

La présente invention vise à apporter ces solutions et à résoudre les problèmes des procédés de l'art antérieur qui ne sont pas naturels, qui perturbent les interventions, qui sont encombrants et encombrent le champ opératoire, qui sont artificiels dans leur utilisation.

Le procédé est maintenant décrit en regard d'une arthroplastie de la hanche, ceci à titre d'exemple uniquement, sans que cela puisse être considéré comme limitatif. Cette description est établie en regard des dessins annexés qui sont représentés sur les parties osseuses représentées de façon schématique, de façon à ne pas encombrer les figures. Ces figures desdits dessins représentent :
- Figure 1 : une vue du dispositif de mise en oeuvre du procédé dans son ensemble,
- Figure 2 : pose d'un premier marqueur sur le bassin,
- Figure 3 : pose d'un deuxième marqueur sur le fémur et acquisition de la position des deux marqueurs,
- Figure 4 : visualisation virtuelle des plans sagittal, frontal et transversal, avec définition d'un repère orthonormé,
- Figure 5 : troisième marqueur associé à des gabarits de têtes fémorales de différents diamètres avec rehausseurs,
- Figure 6 : essai avec une demi-sphère fémorale et son marqueur et acquisition du centre de rotation de la tête fémorale,
- Figure 7 : enregistrement du centre de rotation initial du cotyle au moyen du quatrième marqueur,
- Figure 8 : implantation de la tige fémorale avec un cinquième marqueur posé sur l'implant d'essai, enregistrement et vérification du positionnement de la tête fémorale dans le cotyle,
- Figure 9 : montage de la tête prothétique à la place du cinquième marqueur.

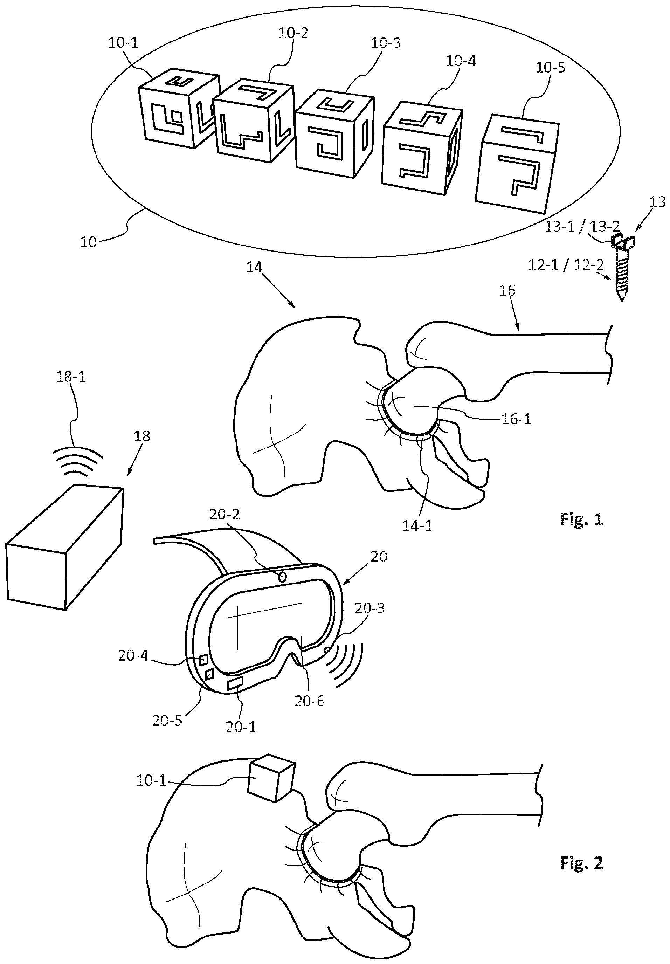

Sur la figure 1, on a représenté le dispositif selon la présente invention qui comprend des marqueurs 10, dans le cas retenu une intervention de Prothèse Totale de Hanche, PHT, 10-1 à 10-5.

Les marqueurs sont les suivants :
- 10-1 : marqueur de référence associé au fémur,
- 10-2 : marqueur de référence associé au bassin,
- 10-3 : marqueur associé à la tête fémorale,
- 10-4 : marqueur associé au cotyle, et
- 10-5 : marqueur de vérification associé à la prothèse fémorale d'essai.

Ces marqueurs sont selon l'invention des QR code 3D, c'est-à-dire des cubes avec chacune des faces de chaque cube identifiée par un code et repérable dans l'espace.

Ces marqueurs 10 sont adaptés à la chirurgie et sont stérilisables et apposables par tout moyen.

Selon des alternatives ne formant pas partie de l'invention revendiquée, d'autres types de marqueurs pourraient être utilisés à condition de pouvoir être repérés en 3D.

On a représenté sur cette même figure 1, des moyens de fixation 12 de ces marqueurs à QR code, sur la partie osseuse, par exemple à l'aide d'une vis chirurgicale 12-1 et 12-2 respectivement pour les marqueurs 10-1 et 10-2, chacune desdites vis étant munie à son extrémité libre de moyens d'encliquetage 13, en l'occurrence 13-1, 13-2, dudit marqueur dans une position unique, notamment en orientation. Pour les autres marqueurs, ils peuvent être directement rapportés sur la prothèse comme cela sera décrit plus avant.

On a schématiquement représenté le bassin 14 et le haut du fémur 16 du patient sur la figure 1.

On a repéré le cotyle en 14-1 et la tête fémorale en 16-1, sains, tels qu'ils se présentent, avant luxation, en faisant abstraction, pour la présente description, des éventuelles zones atteintes d'une quelconque affection.

Le patient est allongé sur la table d'opération en position horizontale, couché sur le côté en décubitus latéral, ce qui permet de définir :
- un plan sagittal PS, horizontal et parallèle au sol,
- un plan transversal PT, c'est-à-dire vertical parallèle au sol, perpendiculaire à l'axe longitudinal du patient, et
- un plan frontal PF, vertical et perpendiculaire aux plans sagittal et frontal.

Ainsi il est possible de définir trois axes X, Y et Z formant un repère orthonormé, ces trois axes étant inclus dans les trois plans précédemment indiqués avec une origine O considérée comme étant le marqueur 10-2 associé au bassin qui est celui de référence.

Il est en outre prévu une unité 18 de traitement logiciel des données et d'enregistrement, munie de moyens d'échanges 18-1 à courte portée, du type Bluetooth^{®}, avec au moins un masque 20 à réalité augmentée, destiné à être porté par le chirurgien durant l'intervention. Le masque peut prendre la forme de lunettes, de casque, ceci étant indépendant de la présente invention.

Chaque masque 20 comprend une source d'énergie autonome 20-1, au moins une caméra 20-2, des moyens d'échange 20-3 à courte portée avec l'unité 18, du type Bluetooth^{®}, un gyroscope 20-4 et un accéléromètre 20-5, et éventuellement d'autres capteurs tels qu'un magnétomètre et un capteur de profondeur de façon à disposer d'une position dans l'espace. Chaque masque 20 comporte également un écran transparent 20-6 sur lequel peuvent apparaître des images et des informations transmises par l'unité 18 de façon à autoriser une superposition de l'image réelle visible en transparence et des images et informations virtuelles affichées sur l'écran.

Selon des alternatives ne faisant pas partie de l'invention revendiquée, les masques peuvent être remplacés par un support du type écran sur lequel est effectuée la superposition mais le masque apporte un confort de travail certain.

Le dispositif est complété par un jeu de n gabarits hémisphériques fémoraux 22, en l'occurrence quatre gabarits hémisphériques fémoraux 22-1 à 22-4. Les diamètres intérieurs sont croissants, ceci de façon connue dans les ancillaires existants.

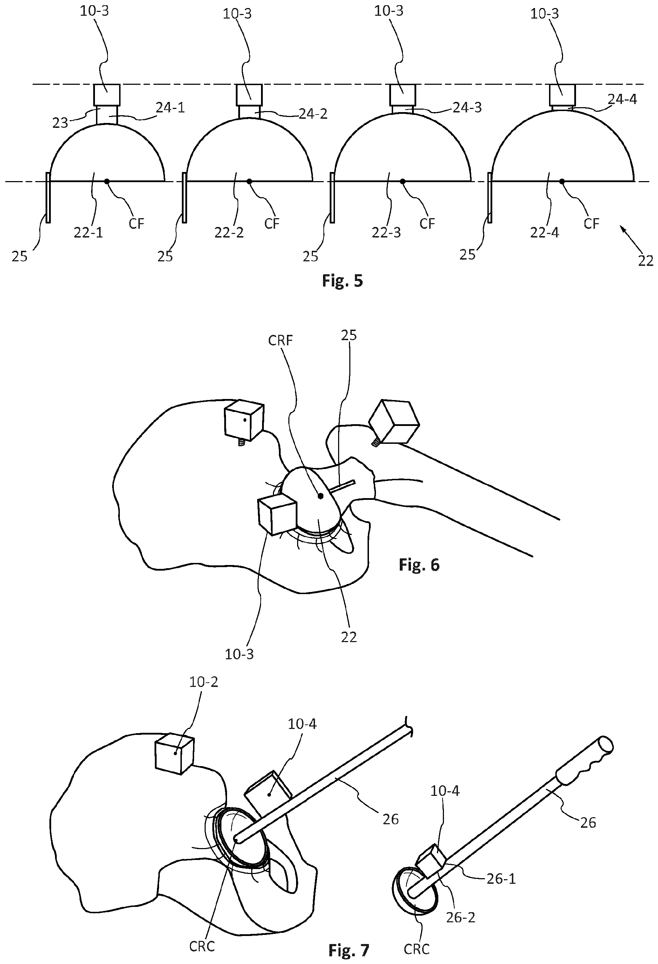

Ces gabarits hémisphériques correspondent à différentes tailles de têtes fémorales et sont destinés à coiffer la tête fémorale à remplacer dans une étape du procédé. La représentation du jeu en est faite en détail sur la figure 5.

Chaque gabarit hémisphérique fémoral 22 est muni de moyens d'encliquetage 23, par exemples identiques aux moyens 13-1, 13-2, destinés à recevoir le marqueur 10-3. Chaque moyen d'encliquetage 23 de chaque gabarit hémisphérique fémoral est associé à une rehausse 24, en l'occurrence quatre rehausses 24-1 à 24-4, adaptées pour être interposées chacune entre le point sommital de l'hémisphère du gabarit hémisphérique fémoral et le marqueur 10-3 à encliqueter. Ainsi, le marqueur 10-3 est toujours à la même distance du centre virtuel CF de l'hémisphère du gabarit hémisphérique fémoral.

Bien entendu, les gabarits peuvent intégrer directement venus de fabrication, les marqueur Chaque gabarit 22 est muni d'une tige 25 d'orientation et la gamme peut correspondre à des diamètres intérieurs de 36, 40, 50 et 62 mm pour donner un exemple chiffré, totalement non limitatif.

Le dispositif est également complété par des cotyles d'essai 24, généralement ceux de l'ancillaire associé à l'intervention, destinés à être fixés sur un manche d'impacteur 26, par exemple à visser. Le manche d'impacteur comprend un support 26-1 de marqueur 10 avec des moyens d'encliquetage 26-2. Le support 26-1 reçoit en l'occurrence le marqueur 10-4 concerné, monté mobile longitudinalement sur le manche pour être positionné en fonction de chaque gabarit cotyloïdien, de façon à déterminer notamment le Centre de Rotation Cotyloïdien, CRC, du cotyle du patient destiné à recevoir la prothèse cotyloïdienne. Ce Centre de Rotation Cotyloïdien est mentionné sur la figure 7.

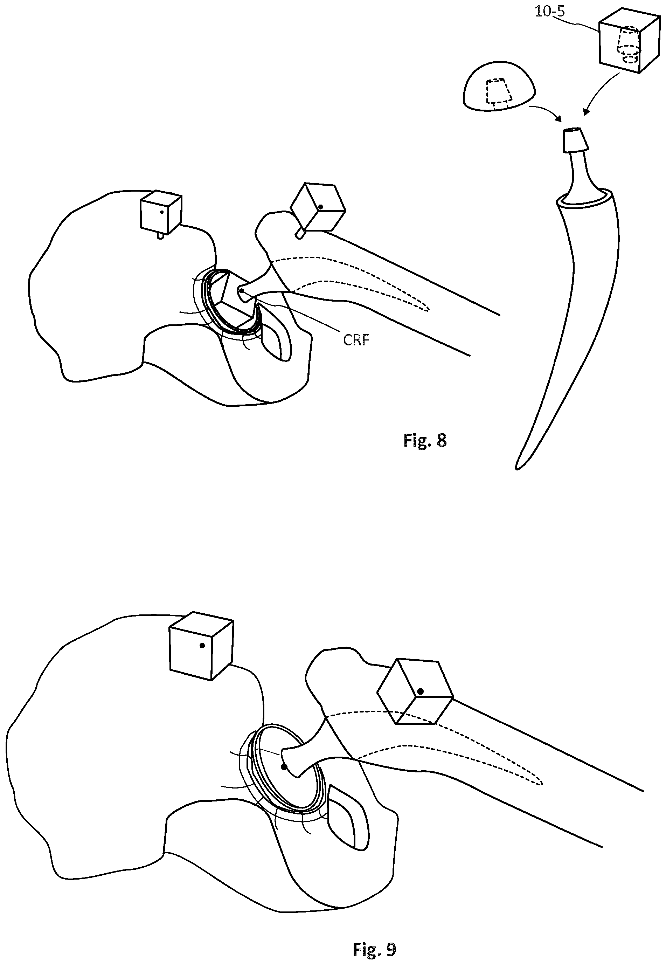

Le dispositif comporte aussi des tiges fémorales d'essais et des râpes fémorales, généralement celles de l'ancillaire avec des têtes fémorales. Le cinquième marqueur 10-5 est avantageusement muni d'une trou comme la tête fémorale de façon à mettre, sur le cône de la tige fémorale d'essai, en lieu et place de la tête fémorale ledit cinquième marqueur, voir figure 8.

Ainsi, le centre du cinquième marqueur 10-5 correspond au centre de rotation de la tête fémorale qui sera reçue sur la tige fémorale définitive qui sera implantée.

Le procédé selon la présente invention consiste en la succession des étapes qui sont maintenant décrites.

Le chirurgien dispose de tout l'ancillaire opératoire nécessaire et connu ainsi que des jeux de prothèses adaptées, tous ces éléments ne faisant pas partie de la présente invention. Seules sont décrites les étapes faisant intervenir le dispositif et le procédé selon la présente invention, à l'exclusion de tous les gestes aseptiques, opératoires, chirurgicaux liés de façon connue au protocole opératoire.

Le chirurgien pose, après incision, la vis chirurgicale 12-1 sur le fémur 16 dans une région proximale de la tête fémorale, ladite vis 12-1 recevant le marqueur 10-1, grâce aux moyens d'encliquetage 13-1. Cette étape est représentée sur la figure 2.

Le marqueur fémoral 10-1 est en position unique et constitue une première position de référence.

Le chirurgien pose une seconde vis chirurgicale 12-2 dans le bassin 14, dans une région proche du cotyle, de façon à ne pas perturber ses propres interventions et gestes. La pose peut être effectuée en passant par l'incision ou en dehors.

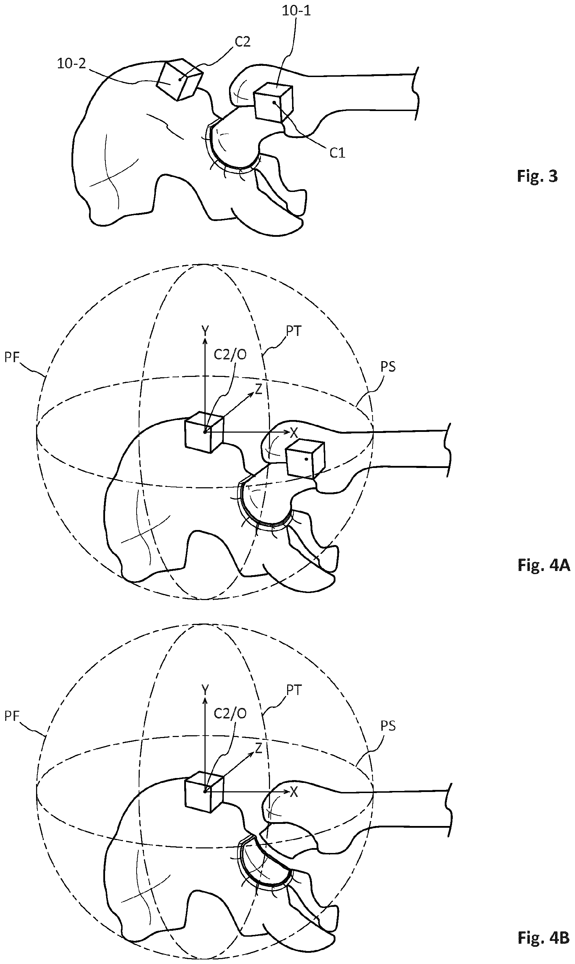

Le deuxième marqueur 10-2 est posé par encliquetage grâce aux moyens 13-2 sur la tête de la seconde vis chirurgicale 12-2. Cette étape est représentée sur la figure 3 qui montre les deux marqueurs tels qu'implantés. C'est le marqueur 10-2 de référence qui constitue le point O du repère orthonormé défini plus avant dans la description.

Ces deux marqueurs 10-1, 10-2 sont reconnus par la caméra 20-2 du masque 20 dès lors que le masque 20 est mis en service et porté par le chirurgien. L'unité 18 d'acquisition des images numériques et de traitement de ces images étant en service également, traite et mémorise les données numériques de ces deux marqueurs.

Il est donc possible de connaître la distance entre les deux centres C1 et C2 des deux marqueurs 10-1 et 10-2. L'unité centrale a positionné les centres C1 et C2 de ces marqueurs en 3D et ces positions sont accessibles et visualisables sur l'écran du masque mais figées.

Le plan sagittal PS est horizontal comme indiqué ci-avant et les plans verticaux considérés sont le plan Transversal PT et le plan Frontal PF. Les trois plans sont perpendiculaires entre eux et passent par le centre du marqueur 10-1, voir figure 4.

Tous les autres marqueurs seront repérés par rapport à ce repère orthonormé.

Ainsi, le chirurgien dispose des mesures et des positions des différents marqueurs avant intervention.

Il reste à déterminer encore des repères mais ils nécessitent que l'articulation de la hanche soit luxée, dans le cas de la voie d'abord retenue, afin d'avoir accès à la partie fémorale, notamment la tête fémorale ainsi qu'à la partie cotyloïdienne.

Le premier repère est celui du centre de rotation fémoral CRF qui doit être déterminé, voir figure 6.

A cet effet le praticien pose, sur la tête fémorale, un des gabarits hémisphériques fémoraux 22-1 à 22-4, celui qui est le plus adapté.

Une fois l'un de ces gabarits retenu, il est laissé en place afin d'acquérir les données nécessaires grâce au marqueur 10-3, disposé sur sa rehausse 24.

Le centre de rotation fémoral CRF est alors déterminé et enregistré en 3D, car connu par rapport au gabarit utilisé. L'unité 18 de traitement logiciel enregistre également ce point CRF dans l'espace.

On note que la tige d'orientation 25 est aligné avec l'axe du col du fémur.

Ce point CRF est référencé par rapport au centre du marqueur 10-1 afin de déterminer la distance entre le centre de rotation fémoral CRF et le centre C1 du marqueur 10-1. Le référencement dans l'espace permet aussi de déterminer l'angle cervico-diaphysaire. L'angle d'antéversion du col du fémur est également enregistré pour apparaître de façon virtuelle si nécessaire, par exemple avec des valeurs angulaires différentielles entre les valeurs enregistrées initialement et les angles enregistrés avec les prothèses d'essais ou définitives.

L'ensemble des informations étant acquis, le chirurgien peut pratiquer la résection de la tête fémorale.

Le chirurgien peut assurer la préparation du fémur pour qu'il reçoive la prothèse fémorale déterminée par les mesures effectuées.

A tout moment le chirurgien dispose des mesures mais surtout des images virtuelles enregistrées des points C1, du Centre de Rotation Fémoral initial, des angles, des axes de façon à positionner la prothèse de façon précise sans avoir besoin de mesurer puisque les valeurs s'affichent.

A cet effet, il est prévu d'équiper soit l'outil, en l'occurrence les râpes, soit la prothèse fémorale d'essai d'un marqueur 10-5 qui est reconnu et repère le nouveau centre de rotation fémoral obtenu avec la prothèse. De plus le Centre de Rotation Fémoral CRF initial est également visible virtuellement par le chirurgien de façon à faire coïncider, avec le moindre écart, les deux centres de rotation initial et de la prothèse ou de prévoir un écart volontairement qui est alors indiqué. Ceci permet au praticien d'ajuster en retirant la prothèse d'essai pour retravailler.

En ce qui concerne le cotyle, les différents cotyles prothétiques d'essais de l'ancillaire, de forme hémisphérique, sont présentés dans le cotyle naturel, afin de déterminer ses dimensions et surtout d'enregistrer le Centre de Rotation Cotyloïdien initial, CRC, naturel, grâce au marqueur 10-4.

Les informations dimensionnelles et d'orientation sont alors déterminées.

Le chirurgien dispose de la distance entre le CRC et le centre C2 du deuxième marqueur 10-2. Les angles d'inclinaison et d'antéversion du cotyle sont déterminés par rapport au repère orthonormé.

Le plan du bord du cotyle ou plan équatorial de l'hémisphère est inclus dans un disque virtuel. Ce disque coupe lesdits plans avec les angles d'inclinaison et d'antéversion déterminés et enregistrés par les moyens unité 18 de traitement logiciel des données et d'enregistrement. Le chirurgien peut alors préparer le cotyle par fraisage notamment pour qu'il puisse recevoir la prothèse cotyloïdienne selon les protocoles connus et envisagés.

La prothèse d'essai cotyloïdienne est présentée avec l'impacteur équipé de son marqueur 10-4 de façon à vérifier les différents paramètres dimensionnels et angulaires associés à la prothèse cotyloïdienne versus les paramètres réels mesurés et enregistrés.

Le chirurgien, en plus des mesures calculées et donc des différentiels qui s'affichent peut constater par lui-même en superposant les points, y compris du centre de la tête fémorale et autre disque équatorial virtuels enregistrés en 3D et la prothèse d'essai en cours de positionnement. Le chirurgien peut à ce stade vérifier, grâce aux projections virtuelles sur les lunettes, la correspondance du nouveau centre de rotation cotyloïdien obtenu après intervention avec le centre de rotation naturel de départ afin de déterminer si le positionnement est acceptable. Si le chirurgien constate au contraire une médialisation ou une latéralisation du centre de rotation cotyloïdien, il est possible de corriger et le chirurgien sait dans quelle sens réaliser ladite correction.

Une fois la préparation finalisée, la prothèse cotyloïdienne est alors impactée et/ou scellée.

La position du cotyle peut aussi être vérifiée à ce stade afin de déterminé s'il a été suffisamment impacté et si cela correspond bien au centre de rotation prothétique enregistré. Il est donc possible de procéder à la réduction de la hanche et à une mise en place de la prothèse fémorale dans la prothèse cotyloïdienne. Le centre de rotation "prothétique" doit être aussi proche que possible du centre de rotation naturel enregistré.

Il est aussi possible de prévoir un second repère orthonormé avec son centre à partir du marqueur 10-1 de fémur avant intervention.

Le chirurgien peut alors superposer le repère orthonormé déterminé à partir du marqueur 10-1 après intervention avec le repère orthonormé figé et enregistré avant intervention, ce qui aide le chirurgien à positionner le membre inférieur après intervention exactement dans la même géométrie qu'initialement.

## Revendications

1. Dispositif pour la mise en oeuvre d'un procédé d'assistance à la pose d'une prothèse entre au moins deux os pour une arthroplastie de hanche, de genoux ou d'épaule, le dispositif comprenant au moins :
- deux marqueurs 3D, dont des marqueurs de référence (10-1, 10-2) destiné chacun à être apposé sur l'un des os,
chaque marqueur étant un QR code 3D, c'est-à-dire un cube dont chacune des faces est identifiée par un code,
- des moyens de fixation des marqueurs de référence sur les os comportant, pour chaque marqueur de référence, une vis chirurgicale munie à son extrémité libre de moyens d'encliquetage dudit marqueur dans une position unique ,
- une unité (18) de traitement logiciel des données et d'enregistrement,
- un masque (20) comprenant :
∘ une source d'énergie autonome (20-1),
∘ une caméra (20-2),
∘ des moyens d'échange (20-3) à courte portée avec ladite unité (18),
∘ un écran transparent (20-6) d'affichage des images et des informations transmises par ladite unité (18) de façon à autoriser une superposition de l'image réelle visible en transparence et des images et informations virtuelles affichées sur l'écran ;
la caméra (20-2) étant agencée pour, lorsque le masque est mis en service, reconnaître les marqueurs de référence (10-1, 10-2), l'unité (18) étant agencée pour déterminer et enregistrer la position en 3D des centres (C1, C2) de ces marqueurs de référence et pour positionner et pour figer, sur l'écran transparent (20-6), les centres (C1, C2) de ces marqueurs de référence, le centre de l'un de marqueurs de référence constituant l'origine (O) d'un repère orthonormé .

2. Dispositif selon la revendication 1, **caractérisé en ce qu'**il comporte un troisième marqueur (10-3, 10-4, 10-5) destiné à être apposé sur une prothèse d'essai ou un outil de préparation, ce troisième marqueur étant un QR code 3D, c'est-à-dire un cube dont chacune des faces est identifiée par un code et **en ce que** la caméra (20-2) est agencée pour reconnaître le troisième marqueur (10-3, 10-4, 10-5), l'unité (18) étant agencée pour déterminer un centre de rotation de la prothèse (CRF, CRC) et pour positionner, sur l'écran transparent (20-6), le centre de rotation de la prothèse dans le repère orthonormé.

3. Dispositif selon la revendication 1 ou 2, **caractérisé en ce que** les paramètres virtuels enregistrés et calculés sont affichés directement sur un écran transparent de façon à les superposer sur chaque membre et chaque prothèse.

4. Dispositif selon l'une quelconque des revendications précédentes, **caractérisé en ce que** les paramètres virtuels enregistrés et calculés sont affichés directement sur un écran transparent monté sous forme d'une paire de lunettes portée par ledit chirurgien.

5. Dispositif selon l'une des revendications précédentes, **caractérisé en ce qu'**il comprend un gyroscope (20-4) et un accéléromètre (20-5), tous deux associés au masque (20) de façon à disposer d'une position dans l'espace.

6. Dispositif selon la revendication 5, **caractérisé en ce qu'**il comprend de plus un magnétomètre et un capteur de profondeur.

## Patentansprüche

1. Vorrichtung zur Ausführung eines Verfahrens zur Unterstützung des Einpassens einer Prothese zwischen mindestens zwei Knochen für eine Hüft-, Knie- oder Schulterarthroplastik, die Vorrichtung mindestens umfassend:
- zwei 3D-Marker, darunter Referenzmarker (10-1, 10-2), die jeweils dazu bestimmt sind, an einem der Knochen angebracht zu werden,
wobei jeder Marker ein 3D-QR-Code ist, also ein Würfel, dessen Seiten jeweils durch einen Code identifiziert werden,
- Mittel zum Befestigen der Referenzmarker an den Knochen, die für jeden Referenzmarker eine chirurgische Schraube aufweisen, die an ihrem freien Ende mit Mitteln zum Einrasten des Markers in einer eindeutigen Position versehen ist,
- eine Einheit (18) zur softwaregestützten Datenverarbeitung und Speicherung,
- eine Maske (20), umfassend:
o eine autonome Energiequelle (20-1),
o eine Kamera (20-2),
o Mittel (20-3) zum Austausch mit der Einheit (18) über eine kurze Reichweite,
o einen transparenten Bildschirm (20-6) zum Anzeigen der von der Einheit (18) übertragenen Bilder und Informationen derart, dass eine Überblendung des transparent sichtbaren realen Bildes und der auf dem Bildschirm angezeigten virtuellen Bilder und Informationen möglich ist;
wobei die Kamera (20-2) so angeordnet ist, dass sie, wenn die Maske in Betrieb genommen wird, die Referenzmarker (10-1, 10-2) erkennt, wobei die Einheit (18) so eingerichtet ist, dass sie die 3D-Position der Zentren (C1, C2) dieser Referenzmarker bestimmt und abspeichert und die Zentren (C1, C2) dieser Referenzmarker auf dem transparenten Bildschirm (20-6) positioniert und einfriert, wobei das Zentrum eines der Referenzmarker den Ursprung (O) eines orthonormierten Koordinatensystems bildet.

2. Vorrichtung nach Anspruch 1, **dadurch gekennzeichnet, dass** sie einen dritten Marker (10-3, 10-4, 10-5) aufweist, der dazu bestimmt ist, an einer Testprothese oder einem Präparationswerkzeug angebracht zu werden, wobei dieser dritte Marker ein 3D-QR-Code ist, also ein Würfel, bei dem jede der Seiten durch einen Code identifiziert wird, und dass die Kamera (20-2) so angeordnet ist, dass sie den dritten Marker (10-3, 10-4, 10-5) erkennt, wobei die Einheit (18) so eingerichtet ist, dass sie ein Rotationszentrum der Prothese (CRF, CRC) bestimmt und das Rotationszentrum der Prothese auf dem transparenten Bildschirm (20-6) in dem orthonormalen Koordinatensystem positioniert.

3. Vorrichtung nach Anspruch 1 oder 2, **dadurch gekennzeichnet, dass** die abgespeicherten und berechneten virtuellen Parameter direkt auf einem transparenten Bildschirm angezeigt werden, so dass sie auf jede Gliedmaße und jede Prothese überblendet werden.

4. Vorrichtung nach einem der vorstehenden Ansprüche,
**dadurch gekennzeichnet, dass** die abgespeicherten und berechneten virtuellen Parameter direkt auf einem transparenten Bildschirm angezeigt werden, der in Form einer Brille angebracht ist, die von dem Chirurgen getragen wird.

5. Vorrichtung nach einem der vorstehenden Ansprüche,
**dadurch gekennzeichnet, dass** sie ein Gyroskop (20-4) und einen Beschleunigungsmesser (20-5) umfasst, die beide so der Maske (20) zugeordnet sind, dass sie über eine Position im Raum verfügen.

6. Vorrichtung nach Anspruch 5, **dadurch gekennzeichnet, dass** sie zusätzlich einen Magnetometer und einen Tiefensensor umfasst.

## Claims

1. A device for implementing a method of assisting the fitting of a prosthesis between at least two bones for hip, knee or shoulder arthroplasty, the device comprising at least
- two 3D markers, including reference markers (10-1, 10-2), each intended to be affixed to one of the bones,
each marker is a 3D QR code, that is, a cube each face of which is identified by a code,
- means for attaching the reference markers to the bones, comprising, for each reference marker, a surgical screw provided at its free end with means for locking said marker in a single position,
- a software data processing and recording unit (18),
- a mask (20) comprising:
∘ a self-contained power source (20-1),
∘ a camera (20-2),
∘ short-range means of exchange (20-3) with said unit (18),
∘ a transparent screen (20-6) for displaying the images and information transmitted by said unit (18) so as to enable superimposition of the real image visible in transparency and the virtual images and information displayed on the screen;
the camera (20-2) being arranged, when the mask is switched on, to recognize the reference markers (10-1, 10-2), the unit (18) being arranged to determine and record the 3D position of the centers (C1, C2) of these reference markers and to position and freeze, on the transparent screen (20-6), the centers (C1, C2) of these reference markers, the center of one of the reference markers constituting the origin (O) of an orthonormal frame of reference.

2. The device according to claim 1, **characterized in that** it comprises a third marker (10-3, 10-4, 10-5) intended to be affixed to a trial prosthesis or preparation tool, this third marker being a 3D QR code, that is, a cube each face of which is identified by a code, and **in that** the camera (20-2) is arranged to recognize the third marker (10-3, 10-4, 10-5), the unit (18) being arranged to determine a center of rotation of the prosthesis (CRF, CRC) and to position, on the transparent screen (20-6), the center of rotation of the prosthesis in the orthonormal reference frame.

3. The device according to claim 1 or 2, **characterized in that** the virtual parameters recorded and calculated are displayed directly on a transparent screen so as to superimpose them on each limb and each prosthesis.

4. The device according to any of the preceding claims,
**characterized in that** the virtual parameters recorded and calculated are displayed directly on a transparent screen mounted in the form of a pair of glasses worn by said surgeon.

5. The device according to one of the preceding claims,
**characterized in that** it comprises a gyroscope (20-4) and an accelerometer (20-5), both associated with the mask (20) so as to provide a position in space.

6. The device according to claim 5, **characterized in that** it further comprises a magnetometer and a depth sensor.
